# EUROPEAN PATENT APPLICATION

(11) **EP 3 903 589 A2**
(43) Date of publication of application: **03.11.2021**
(21) Application number: 19905385.1
(22) Date of filing: 27.12.2019
(51) Int. Cl.: A23B 7/10, A23L 19/20, A23L 33/105

(54) **KIMCHI FOR PREVENTING OR TREATING HELICOBACTER PYLORI-ASSOCIATED DISEASES**

(30) Priority: 28.12.2018 KR 20180171845
(71) Applicant: CJ Cheiljedang Corporation, Seoul 04560 (KR)
(72) Inventor: LEE, Dong Yun, Hwaseong-si, Gyeonggi-do 18503 (KR); CHOI, Seung Hye, Yongin-si, Gyeonggi-do 16833 (KR); OH, Ji Young, Yongin-si, Gyeonggi-do 16949 (KR)
(74) Representative: Cabinet Laurent & Charras
(86) International application number: PCT/KR2019/018606
(87) International publication number: WO 2020/139020

(57) **Abstract**

The present application relates to kimchi containing lactic acid bacteria which can be effectively used for the prevention and treatment of *Helicobacter* pylori-associated diseases.

## Description

### Technical Field

The present application relates to kimchi containing lactic acid bacteria which can be effectively used for the prevention and treatment of a *Helicobacter* pylori-associated disease.

### BACKGROUND ART

In 1983, Australian microbiologists Marchall and Warren first discovered a curved bacterium in gastric mucosal biopsy tissues in patients with type B gastritis, and then cultivated the bacterium and identified that it was a new species associated with *Campylobacter* genus. Since then, active research has been conducted on the association between *Helicobacter pylori* and upper gastrointestinal diseases such as gastritis, peptic ulcer, and the like. *Helicobacter pylori* is a Gram-negative bacillus, has flagella surrounded by a thin membrane, releases a large amount of urease, and is known to be spread through a fecal-oral route, and is a pathogen that has attracted great interest worldwide because the World Health Organization (WHO) identified it as a definite carcinogen in 1994.

Currently, the pathogenesis of a disease caused by *Helicobacter pylori* is known as follows: bacteria that entered the gastric lumen along with food penetrate the gastric mucus layer by flagella having strong motility, and inhabit the upper epithelial layer and the junction between cells in the gastric mucus layer, and release a large amounts of urease to change the mucus layer to alkaline, thereby leading to abnormally excessive stimulation of gastric acid secretion by gastrin, and in turn, resulting in inflammation and an ulcer. In addition, according to a recent study that found that *Helicobacter pylori* is an important risk factor for the pathogenesis of gastric adenocarcinoma, the World Health Organization has designated *Helicobacter pylori* as a group I carcinogen.

In particular, infection with *Helicobacter pylori* has been reported to occur frequently in developing countries including South Korea. According to the national epidemiological survey conducted on 5,732 people with no upper gastrointestinal tract symptoms, the overall prevalence of *Helicobacter pylori* infection was 46.6%, and the infection rate was 69.4% in adults over 16 years old and was especially high when people were aged in their 40s (78.5%). In addition, according to the investigation conducted on 1,031 patients with peptic ulcer, 66% of gastric ulcer patients and 79% of duodenal ulcer patients were found to be infected with *Helicobacter pylori* and 71% of patients with gastric and duodenal ulcers were reported to be infected with *Helicobacter pylori.*

As a method for treating the above diseases caused by *Helicobacter pylori* infection, currently, chemotherapy with antibiotics is mostly performed. However, the widespread use of this method may be effective in the short term, but this method has a problem that involves a risk of the emergence of antibiotic resistant strains due to the continued use of antibiotics. In addition, the continued use of antibacterial therapy has been reported to cause various side effects, such as diarrhea, reflux esophagitis, suppression of the normal flora in the intestine, the emergence of resistant strains, and the like.

Therefore, there is a need for the development of a suitable antimicrobial material or food which can suppress the *Helicobacter pylori,* while replacing the therapeutic agent causing the above-described side effects and resistance in the human body.

Meanwhile, fermented lactic acid bacteria of kimchi, which is a traditional fermented food, are GRAS (Generally Recognized As Safe) grade bacteria, which has long been consumed with fermented foods by humans. Due to the advantages of the functionality and safety of lactic acid bacteria, attention is focused on the development of functional foods and drugs using the lactic acid bacteria. For example, the present applicants have reported *Leuconostoc mesenteroides* CJLM119 strain producing a reduced amount of gas and a method for preparing kimchi using the same, and *Lactobacillus plantarum* CJLP133 strain and a composition for treating intestinal diseases and enhancing immunity comprising the same (Patent Documents 1 to 3). However, studies on methods for preventing or treating a disease caused by *Helicobacter pylori* infection through fermented foods such as kimchi which are frequently consumed are not complete, and are still in progress.

**Prior art documents**

**Patent Documents**
(Patent Document 1) Korean Patent No. 1,807,995
(Patent Document 2) Korean Patent No. 1,486,999
(Patent Document 3) Korean Patent No. 1,406,168

### DISCLOSURE OF THE INVENTION

### TECHNICAL PROBLEM

The present application relates to kimchi containing lactic acid bacteria having an inhibitory effect on *Helicobacter pylori* as an active ingredient, and a use of the kimchi and an extract thereof for use in the prevention and treatment of a *Helicobacter* pylori-associated disease.

### TECHNICAL SOLUTION

In order to solve the above problem, one aspect of the present application provides kimchi including *Leuconostoc mesenteroides* CJLM119 and *Lactobacillus plantarum* CJLP133, which are kimchi-derived lactic acid bacteria, and a method for preparing the same.

Another aspect of the present application provides a composition for ameliorating a *Helicobacter* pylori-associated disease, the composition including the kimchi or an extract thereof.

A still another aspect of the present application provides a method for preventing, treating, or ameliorating a *Helicobacter* pylori-associated disease, the method using the kimchi or an extract thereof.

### ADVANTAGEOUS EFFECTS

The kimchi including *Leuconostoc mesenteroides* CJLM119 and *Lactobacillus plantarum* CJLP133, which are kimchi-derived lactic acid bacteria, or an extract thereof according to the present application has an inhibitory activity against *Helicobacter pylori,* and upon prolonged administration, can prevent, treat, or delay the progression of atrophic gastritis and gastric ulcer caused by *Helicobacter pylori,* and can also be usefully used for the prevention or treatment of gastric cancer caused by *Helicobacter pylori.*

However, effects of the present application are not limited to the above-mentioned effects and other effects not mentioned will be clearly understood from the following description by those skilled in the technical field to which the present invention pertains.

### BRIEF DESCRIPTION OF THE DRAWINGS

FIG. 1 is a graph showing the inhibitory effect of kimchi of the present application on the *Helicobacter pylori* bacteria.
FIG. 2 is an electrophoresis picture showing the effect of the kimchi of the present application on the expression of various vascular growth factors.
FIG. 3 is an electrophoresis picture showing the effect of the kimchi of the present application on the expression of various inflammatory factors.
FIG. 4 is a graph showing the effect of the kimchi of the present application on the tight junctions of cells in a *Helicobacter pylori* infection model.
FIG. 5 is a graph showing the effect of the kimchi of the present application on the growth of a gastric cancer cell line.
FIG. 6 is a graph showing the effect of the kimchi of the present application on the apoptosis of a gastric cancer cell line.
FIG. 7 is an electrophoresis picture showing the effect of the kimchi of the present application on the expression of various apoptosis factors of a gastric cancer cell line.
FIG. 8 is an electrophoresis picture showing the effect of the kimchi of the present application on the expression of cell cycle-associated factors of a gastric cancer cell line.
FIG. 9 shows the design of animal experiment to determine the effect of the kimchi of the present application on a *Helicobacter* pylori-associated disease.
FIG. 10 is a graph showing the effect of the kimchi of the present application on the gross lesion index score in a *Helicobacter* pylori-infected animal model.
FIG. 11 is a photograph showing the effect of the kimchi of the present application on the development of gastric cancer in a *Helicobacter* pylori-infected animal model.

### MODE FOR CARRYING OUT THE INVENTION

Hereinafter, the present application will be described in detail.

### 1. Kimchi including Leuconostoc mesenteroides CJLM119 and Lactobacillus plantarum CJLP133 and a method for preparing the same

The present application provides kimchi including *Leuconostoc mesenteroides* CJLM119 and *Lactobacillus plantarum* CJLP133 and a method for preparing the same.

The *Leuconostoc mesenteroides* CJLM119 is the microbial strain deposited at the Korea Research Institute of Bioscience and Biotechnology Gene Bank under the accession number KCTC 13043BP, and a method for isolation and identification of the strain is specifically disclosed in Korean Patent No. 1,807,995. In addition, the *Lactobacillus plantarum* CJLP133 is the microbial strain deposited at the Korea Research Institute of Bioscience and Biotechnology Gene Bank under the accession number KCTC 11403BP, and a method for isolation and identification of the strain is specifically disclosed in Korean Patent No. 1,486,999.

In the present application, "kimchi" is meant to include, without limitation, foods prepared by salting and seasoning vegetables and then fermenting the same. In one embodiment of the present application, the vegetable may be Kimchi cabbages, white radishes, spring onions, mustard leaves, cucumbers, and the like. Specifically, the vegetable may be Kimchi cabbages, and any other vegetables may be used as raw materials of kimchi that is the subject of the present application. In one embodiment of the present application, the vegetable may be, but is not limited to, a vegetable having a high content of lycopene. In another embodiment of the present application, the lycopene may be included in a content of 0.05 to 5 mg, such as 0.1 to 3 mg, 0.2 to 2 mg, 0.3 to 1 mg, 0.5 to 0.8 mg per 100 g of the vegetables, but the content is not limited thereto.

The vegetable having a high content of lycopene may be Kimchi cabbage, and specifically, Kimchi cabbage using the Kimchi cabbage variety of TheHanlho (Variety Application Publication No. 2015-688, published on 15 December 2015). When the vegetables having a high content of lycopene are fermented with *Leuconostoc mesenteroides* CJLM119 and *Lactobacillus plantarum* CJLP133, the decrease in the lycopene content is prevented, and thus the cancer prevention function of lycopene can be maintained.

In one embodiment of the present application, the seasoning is prepared by mixing ingredients such as red pepper powders, garlic, ginger, salted fish, etc., and in addition to these, for the ingredients constituting the seasoning, any other ingredients can be added or excluded as appropriate, if necessary depending on personal preferences, fermentation methods, fermentation period, etc.

In one embodiment of the present application, the kimchi of the present application may be prepared by a method of mixing the salted vegetables, for example, the salted Kimchi cabbages, with the seasoning and then fermenting the same, according to a conventional method for preparing kimchi. As the vegetable, it may be more appropriate to use a vegetable having a high content of lycopene. However, the present application is not limited thereto, and conventional vegetables may be used without limitation for the preparation of the kimchi of the present application.

In another embodiment of the present application, the kimchi of the present application may further include known additives which are acceptable for foods. As the additives, various natural fragrances such as plum fragrance, lemon fragrance, pine apple fragrance, herb fragrance or the like; various natural pigments such as natural fruit juice, chlorophyllin, flavonoids or the like; sweetening components such as fructose, honey, sugar alcohol, or sugar; or an acidulant such as citric acid or sodium citrate may be included without limitation in the kimchi of the present application.

In one embodiment of the present application, the kimchi of the present application may include 0.01 to 5 wt%, specifically 0.02 to 3 wt%, more specifically 0.05 to 2.0 wt% of the *Leuconostoc mesenteroides* CJLM119, based on the total weight of the kimchi.

In another embodiment of the present application, the kimchi of the present application may include 0.01 to 5 wt%, specifically 0.02 to 3 wt%, more specifically 0.05 to 2.0 wt% of the *Lactobacillus plantarum* CJLP133, based on the total weight of the kimchi.

In one embodiment of the present application, the kimchi of the present application may be prepared by adding 0.01 to 5 wt%, specifically 0.02 to 3 wt%, more specifically 0.05 to 2.0 wt% of the *Leuconostoc mesenteroides* CJLM119 and 0.01 to 5 wt%, specifically 0.02 to 3 wt%, more specifically 0.05 to 2.0 wt% of the *Lactobacillus plantarum* CJLP133 to the salted vegetables, for example, the salted vegetables having a high content of lycopene, and then performing fermentation at a specific temperature for a specific period of time.

The temperature during fermentation is not particularly limited, but the fermentation may be performed, for example, at 1°C to 30°C, specifically 3°C to 15°C, or 5°C to 10°C.

The fermentation period is not particularly limited, but the fermentation may be performed, for example, for 1 to 365 days, specifically 5 to 180 days, 10 to 90 days, 15 to 60 days, or 20 to 40 days.

In one embodiment of the present application, the *Leuconostoc mesenteroides* CJLM119 and the *Lactobacillus plantarum* CJLP133 strains themselves may be used, but a culture including the strains may also be used. In one embodiment of the present application, the *Leuconostoc mesenteroides* CJLM119 and the *Lactobacillus plantarum* CJLP133 strains may be used as live cells, dead cells, or mixtures thereof.

In the present application, the term "culture" means a material resulting from culture of the *Leuconostoc mesenteroides* CJLM119 and the *Lactobacillus plantarum* CJLP133 strains after inoculation into medium. For example, the culture may include a culture itself obtained by culturing the *Leuconostoc mesenteroides* CJLM119 and the *Lactobacillus plantarum* CJLP133 strains in the medium (that is, the culture may include the *Leuconostoc mesenteroides* CJLM119 and the *Lactobacillus plantarum* CJLP133 strains, the medium or metabolic products of the strains), a filtrate (e.g., a centrifuged supernatant) obtained by filtering or centrifuging the culture to remove the strains, or the like. In addition, the culture of the present application may include, without limitation, one obtained by drying (e.g., freeze-drying) and powdering the culture.

In one embodiment of the present application, culturing the strains may be performed at a temperature of 10°C to 30°C for 6 to 48 hours, specifically at a temperature of 20°C to 30°C for 12 to 36 hours or 18 to 30 hours. But the present application is not limited thereto.

As the medium, any known medium for lactic acid bacteria may be used without limitation. The medium may include a carbon source and a nitrogen source. The carbon source may include one or more of sucrose, glucose, fructose, and the like, and the nitrogen source may include one or more of, but is not limited to, yeast extract, peptone, beef extract, malt extract, corn steep liquor, ammonium citrate, ammonium sulfate, ammonium chloride, ammonium phosphate, ammonium carbonate, ammonium nitrate, and the like. In addition, the medium may further include one or more of Tween 80, sodium citrate, potassium phosphate, sodium acetate, manganese sulfate, magnesium sulfate, and distilled water.

### 2. A composition for ameliorating a Helicobacter pylori-associated disease, including the kimchi of the present application or an extract thereof

The present application provides a composition for ameliorating a *Helicobacter* pylori-associated disease, the composition including kimchi including *Leuconostoc mesenteroides* CJLM119 and *Lactobacillus plantarum* CJLP133 or an extract thereof.

Kimchi of the present application and a method for preparing the same are the same as those mentioned in "1. **Kimchi including *Leuconostoc mesenteroides* CJLM119** and ***Lactobacillus* plantarum CJLP133 and a method for preparing the same,"** and thus, the description thereof is omitted.

In the present application, the *Helicobacter pylori-*associated disease may include, but is not limited to, gastritis, gastric ulcer, gastric cancer, and the like.

In the present application, "kimchi extract" refers to a material extracted by any method from kimchi, which is a raw material, and is meant to include, without limitation, all of an extract thus extracted, a concentrate obtained therefrom, a dried product and powders of the concentrate.

When the extract is obtained by extracting kimchi, which is a raw material, conventional extraction methods, such as solvent extraction, ultrasonic extraction, filtration and reflux extraction, may be used without limitation. Specifically, solvent extraction or reflux extraction may be used, but the present application is not limited thereto. The extraction process may be repeated several times, after which the extract may be further subjected to steps such as concentration or freeze-drying. Specifically, high concentration kimchi extract powders may be prepared by concentrating the obtained kimchi extract under reduced pressure so as to obtain a concentrate, and freeze-drying the concentrate, and using a grinder.

In one embodiment of the present application, the kimchi extract is meant to include, without limitation, an extract obtained by extracting kimchi with water, an organic solvent, or a mixed solvent thereof, a concentrate or powders of the extract. The organic solvent may be any one selected from the group consisting of alcohols, specifically C₁-C₄ lower alcohols, such as methanol, ethanol, propanol, or butanol, hexane (n-hexane), ethers, glycerol, propylene glycol, butylene glycol, ethyl acetate, methyl acetate, dichloromethane, chloroform, benzene and a mixed solvent thereof. Specifically, the extraction solvent may be any one selected from the group consisting of water, a C₁-C₄ alcohol, and a mixed solvent of the C₁-C₄ alcohol and water. For example, the extraction solvent may be an aqueous methanol solution or an aqueous ethanol solution. The aqueous solution of methanol or ethanol may be 10% (v/v) to 99% (v/v) of an aqueous solution, specifically 20% (v/v) to 70% (v/v) of an aqueous solution, and more specifically, 30% (v/v) to 60% (v/v) of an aqueous solution, but the present application is not limited thereto.

In one embodiment of the present application, the extraction may be carried out at a temperature of 5°C to 100°C, specifically at a temperature of 20°C to 95°C, and more specifically at a temperature of 50°C to 90°C, but the present application is not limited thereto. In addition, the extraction may be carried out for 1 to 20 hours, specifically for 2 to 16 hours, and more specifically for 3 to 12 hours, but the present application is not limited thereto.

In one embodiment of the present application, the extraction may be carried out once to eight times, specifically once to six times, and more specifically once to five times, but the present application is not limited thereto. The extract may be a single extract obtained from each extraction or a mixed extract of extracts obtained from each extraction, specifically a mixed extract of extracts obtained from each extraction.

In the present application, the term "amelioration" refers to, without limitation, all actions that at least decrease parameters, such as a degree of symptom, associated with a treatment by administration of the kimchi of the present application or the extract thereof. The amelioration of the *Helicobacter* pylori-associated disease of the present application may be an effect of the *Leuconostoc mesenteroides* CJLM119 and the *Lactobacillus plantarum* CJLP133 themselves, and may be an effect caused by fermentation of the vegetables by the strains, but the mechanism is not limited thereto.

In the present application, the composition may be a health functional food.

The term "health functional food" is the same term as a food for special health use (FoSHU) and refers to a food which is processed to effectively exert a body-regulating function in addition to nutrient supply, thus having high medicinal and medical effects. Here, the term "function(al)" means pertaining to controlling nutrients for structures and functions of the human body or obtaining beneficial effects in hygienic use such as in physiological functions.

The health food composition can be prepared using a method conventionally used in the art. Materials and ingredients conventionally used in the art may be used for the preparation of the health food composition. In addition, the health food composition may be prepared into any formulation that is regarded as a food, without limitations. The health food composition may be prepared into various forms of formulations and has an advantage over general drugs in that the health food composition is free of side effects which might occur upon long-term intake of drugs because it is based on food materials. Further, the health food composition is of high portability such that it can be ingested as an aid for promoting an ameliorative effect on the symptoms of the *Helicobacter* pylori-associated disease.

The health food refers to a food having an active health maintenance or promotion effects as compared with a general food, and a health supplement food refers to a food for health supplement purposes. The terms functional food, health food, and health supplement food are interchangeably used, as the case may be.

In the present application, the composition of the present application may further include a physiologically acceptable carrier. A type of the carrier is not particularly limited, and any carrier may be used without limitation as long as it is commonly used in the art. Further, the composition of the present application may include additional ingredients that can be commonly used in food compositions to improve odor, taste, visual appearance, and the like. For example, the composition of the present application may include vitamin A, vitamin C, vitamin D, vitamin E, vitamin B1, vitamin B2, vitamin B6, vitamin B12, niacin, biotin, folate, pantothenic acid, and the like. In addition, the composition of the present application may include minerals such as zinc (Zn), iron (Fe), calcium (Ca), chromium (Cr), magnesium (Mg), manganese (Mn), copper (Cu), and the like, and amino acids such as lysine, tryptophan, cysteine, valine, and the like.

In addition, the composition of the present application may include food additives such as preservatives, sterilizing agents, antioxidants, colorants, color fixing agent, bleaches, seasonings, sweeteners, flavoring agents, swelling agents, reinforcing agents, emulsifiers, thickeners, coatings, foam inhibitors, solvents, improving agents, etc. The additive may be selected according to the type of a food and used in an appropriate amount. The proportion of such additive is not critical, but it is common to select the proportion of such additive in the range of 0.01 to 5 parts by weight per 100 parts by weight of the composition of the present application.

The content of kimchi or the extract thereof contained in the composition of the present application may be appropriately determined depending on the purpose of use. In one embodiment of the present application, the content of the kimchi or the extract thereof contained in the composition of the present application may be, but is not limited to, 0.0001 to 20.0 wt%, for example, and specifically 0.001 to 1.0 wt%, based on the total weight of the composition. When the content of the kimchi or the extract thereof is in the above range, it is possible to obtain the ameliorative effect on the symptoms of the *Helicobacter* pylori-associated disease with an economical amount. However, the amount of the kimchi or the extract thereof for the long-term intake for health and hygiene, or health control may be below the above range. Since the kimchi or the extract thereof does not have any safety problems, it may be used in a larger amount than the above range.

The composition of the present application may further include ingredients commonly added in the manufacture of foods, in addition to the kimchi of the present application or the extract thereof , and for example, the composition may include proteins, carbohydrates, fats, nutrients, condiments, flavoring agents, and the like. Examples of such carbohydrates are monosaccharides such as glucose, fructose and the like; disaccharides such as maltose, sucrose, oligosaccharides, and the like; and conventional sugars such as polysaccharides such as dextrin, cyclodextrin, and sugar alcohols such as xylitol, sorbitol, and erythritol. As the flavoring agent, natural flavoring agents such as Thaumatin and extracts of Stevia and synthetic flavoring agents may be used. For example, when the health food composition of the present application is prepared as a drink, citric acid, high fructose corn syrup, sugar, glucose, acetic acid, malic acid, fruit juice, natural extracts, and the like may be further included in addition to the kimchi extract of the present application.

A type of the composition is not particularly limited. Examples of the food to which the kimchi of the present application or the extract thereof can be added are meat, sausages, bread, chocolate, candies, snacks, confectioneries, pizza, ramen, other noodles, gums, dairy products including ice cream, various soups, beverages, teas, drinks, alcoholic beverages, and vitamin complexes, and may include all health foods in their usual sense.

Since the kimchi of the present application or the extract thereof includes the *Leuconostoc mesenteroides* CJLM119 and the *Lactobacillus plantarum* CJLP133, and these two lactic acid bacteria have the feature of highly inhibiting the activity of *Helicobacter pylori,* the composition including the kimchi of the present application or the extract thereof can be usefully used for the prevention or treatment of related diseases caused by *Helicobacter pylori.*

### 3. A method for preventing, treating, or ameliorating a Helicobacter pylori-associated disease, the method using the kimchi of the present application or the extract thereof

The present application provides a method for preventing, treating, or ameliorating a *Helicobacter pylori-*associated disease, comprising the step of administering to a subject having or likely to develop a *Helicobacter* pylori-associated disease the kimchi or the extract thereof.

The present application provides a use of the kimchi or the extract thereof for the prevention, treatment, or amelioration of a *Helicobacter* pylori-associated disease.

The present application provides a use of the kimchi or the extract thereof for the preparation of a composition for preventing, treating, or ameliorating a *Helicobacter* pylori-associated disease.

Kimchi of the present application or an extract thereof and a method for preparing the same are the same as those mentioned in "1. **Kimchi including *Leuconostoc mesenteroides* CJLM119 and *Lactobacillus* plantarum CJLP133 and a method for preparing the same"** and **"2.** A **composition for ameliorating** a ***Helicobacter* pylori-associated disease, including the kimchi of the present application or an extract thereof,"** and thus, the description thereof is omitted.

In the present application, the term "prevention" is meant to include all actions that inhibit or delay a symptom of a *Helicobacter* pylori-associated disease by administration of the kimchi of the present application or the extract thereof.

In addition, in the present application, the term "treatment" is mean to include all actions that improve or beneficially modify a symptom of a *Helicobacter pylori-*associated disease by administration of the kimchi of the present application or the extract thereof.

In addition, in the present application, the term "administration" refers to introducing a predetermined substance into a subject in an appropriate manner.

In addition, in the present application, the term "subject" refers to all animals, such as rats, mice, and livestock, including humans. A specific example thereof may be a mammal including a human. As another example, the subject may be one having or likely to develop a *Helicobacter* pylori-associated disease.

In the method of the present application, the route of administration and the mode of administration, of the kimchi of the present application or the extract thereof, are not particularly limited, and any route of administration and any mode of administration may be followed as long as the composition comprising the kimchi of the present application or the extract thereof can reach a desired target site. Specifically, the kimchi or the extract thereof may be administered via various routes including oral or parenteral routes, but oral administration is common.

In one embodiment of the present application, the composition of cancer prevention kimchi as a food composition for dietary intervention for *Helicobacter pylori* infection was newly studied. In an another embodiment of the present application, it was hypothesized that dietary intervention with cancer preventive kimchi in a mouse model can typically prevent, ameliorate or treat *Helicobacter* pylori-associated diseases such as gastritis, gastric ulcer, gastric cancer, and the like. The cancer preventive kimchi may be prepared by adding the vegetable having a high content of lycopene, the *Leuconostoc mesenteroides* CJLM119 strain, and the *Lactobacillus plantarum* CJLP133 strain in a conventional kimchi preparation method, but the present application is not limited thereto. In one embodiment of the present application, the kimchi according to the present application or the extract thereof shows an excellent prophylactic and therapeutic effect against *Helicobacter* pylori-associated diseases, in particular gastritis, gastric ulcer, and gastric cancer.

According to one embodiment of the present application, the *Leuconostoc mesenteroides* CJLM119 and the *Lactobacillus plantarum* CJLP133 strains, which are the lactic acid bacteria used in the preparation of the kimchi of the present application, have the remarkable inhibitory activity against *Helicobacter pylori,* compared to other strains that belong to the same species.

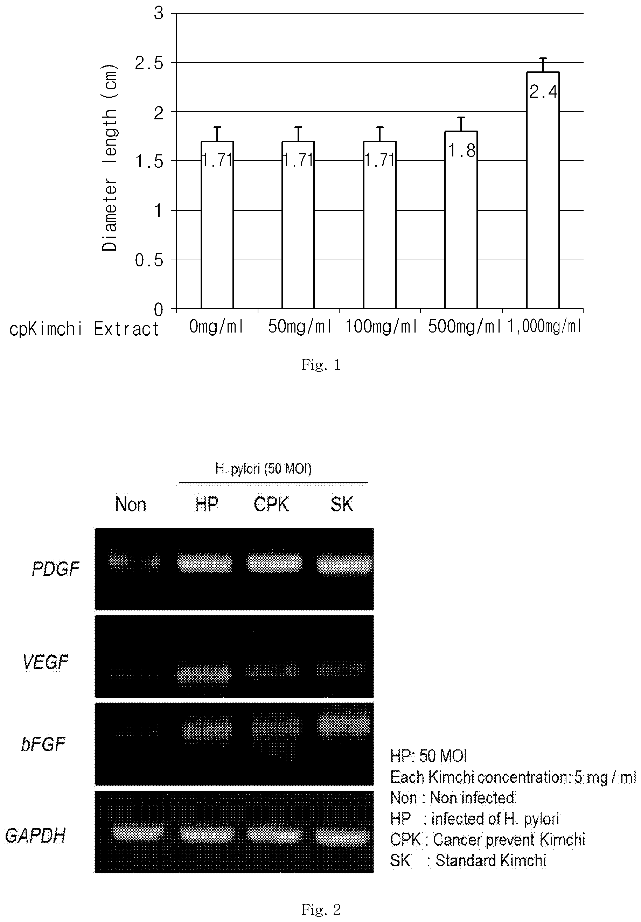

According to another embodiment of the present application, the kimchi including the *Leuconostoc mesenteroides* CJLM119 and the *Lactobacillus plantarum* CJLP133 strains shows the inhibitory effect against *Helicobacter pylori* at a predetermined concentration (see FIG. 1).

According to another embodiment of the present application, the kimchi of the present application can significantly suppress the expressions of vascular growth factors and inflammatory factors in normal gastric cells and restore the tight junctions of the cells (see FIGS. 2 to 4).

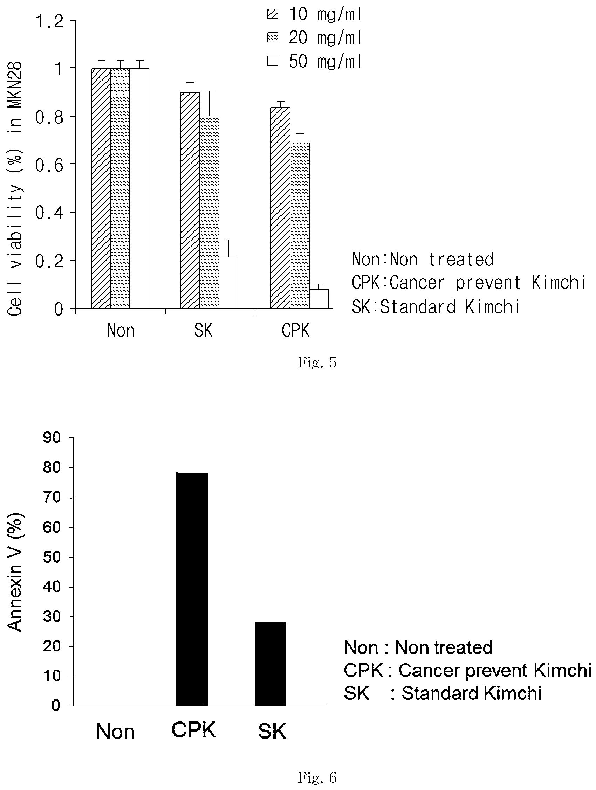

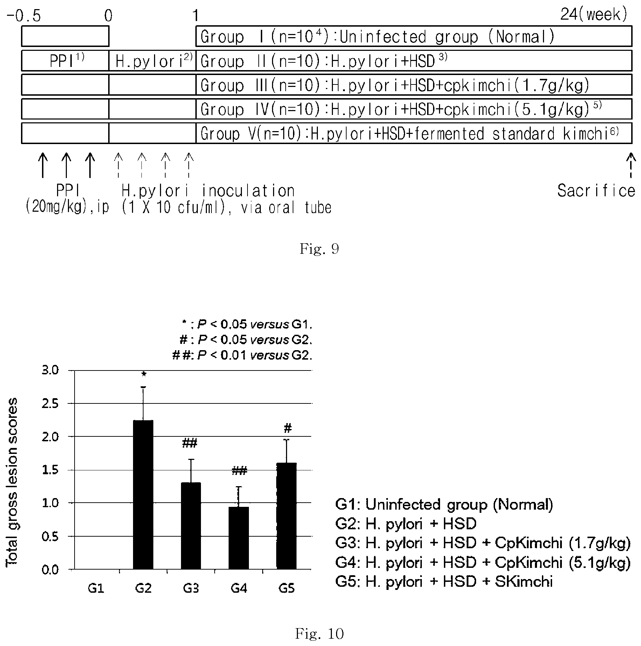

According to another embodiment of the present application, the kimchi of the present application can significantly inhibit the growth of gastric cancer cells, induce apoptosis of gastric cancer cells (see FIGS. 5 and 6), and inhibit the formation of gastric cancer in a *Helicobacter* pylori-infected animal model (see FIG. 10).

As such, the kimchi including the *Leuconostoc mesenteroides* CJLM119 and the *Lactobacillus plantarum* CJLP133 strains of the present application exhibits significant inhibitory activity against *Helicobacter pylori,* and thus, can be usefully used for the prevention, treatment, or amelioration of various diseases that can result from *Helicobacter pylori,* such as gastritis, gastric ulcer, and gastric cancer.

Hereinafter, the present application will be described in detail through Examples.

However, the following Examples merely specifically illustrate the present application, and the contents of the present application are not limited by the following Examples.

First, the preparation process, test method and statistics for the kimchi of the present application will be described. Statistical analyses were conducted with GraphPad Prism (GraphPad Software, La Jolla, CA, USA) and SPSS software (version 12.0; SPSS Inc., Chicago, IL, USA). Statistical significance between groups was determined by Mann-Whitney U test. Statistical significance was accepted at p < 0.05.

### Example 1: Isolation of strains having high growth inhibitory activity against Helicobacter pylori bacteria

### <1-1> Strain isolation and identification

The *Leuconostoc mesenteroides* CJLM119, which is a lactic acid bacterium derived from kimchi and is used for the preparation of the kimchi of the present application, is the microbial strain deposited at the Korea Research Institute of Bioscience and Biotechnology Gene Bank under the accession number KCTC 13043BP, and a method for isolation and identification of the strain is specifically disclosed in Korean Patent No. 10-1807995. In addition, the *Lactobacillus plantarum* CJLP133, which is a lactic acid bacterium derived from kimchi and is used for the preparation of the kimchi of the present application, is the microbial strain deposited at the Korea Research Institute of Bioscience and Biotechnology Gene Bank under the accession number KCTC 11403BP, and a method for isolation and identification of the strain is specifically disclosed in Korean Patent No. 10-1486999. Accordingly, the detailed description thereof is omitted.

In the present application, the kimchi was prepared using the *Leuconostoc mesenteroides* CJLM119 and the *Lactobacillus plantarum* CJLP133 strains according to a conventional preparation method.

### <1-2> Selection of strains having high inhibitory activity against Helicobacter pylori bacteria

The *Helicobacter pylori* inhibitory activities of the *Leuconostoc mesenteroides* CJLM119 and the *Lactobacillus plantarum* CJLP133 strains, which were isolated and identified in Example <1-1>, and *Leuconostoc mesenteroides* KCTC3100 / KCTC3722 and *Lactobacillus plantarum* KCTC3108 /DSM17853, which were used as controls, were measured.

As a result, the *Leuconostoc mesenteroides* CJLM119 and the *Lactobacillus plantarum* CJLP133 strains showed the remarkable inhibitory activity against *Helicobacter pylori,* compared to other strains that were used as controls and belong to the same species.

### Example 2: Preparation of kimchi

Standard kimchi (hereinafter, sometimes referred to as 'sKimchi' or 'SK') which is a control and cancer preventive kimchi (hereinafter, sometimes referred to as 'CpKimchi' or 'CPK') according to the present application were prepared as follows.

### <2-1> Preparation of standard kimchi (sKimchi)

Based on the standardized kimchi recipe of World Institute of Kimchi, standard kimchi was prepared by mixing salted Kimchi cabbages with seasoning including red pepper powders, garlic, ginger, anchovy sauce, ground white radish, spring onion, glutinous rice paste, and sugar, and then performing fermentation at a low temperature.

### <2-2> Preparation of cancer preventive kimchi (CpKimchi)

Cancer preventive kimchi is mainly made from Kimchi cabbage having a high content of lycopene. Seeds of "TheHanlho" (Variety Application Publication No. 2015-688, published on 15 December 2015) which is the Kimchi cabbage variety having a high content of lycopene, which does not exist in normal Kimchi cabbage, were purchased. The Kimchi cabbages were secured through contract farming with Kimchi cabbage farmers and the harvested Kimchi cabbages were used for cancer preventive kimchi after salting according to the kimchi preparation process. Cancer preventive kimchi was prepared in the same manner as in the preparation of standard kimchi, except that: the Kimchi cabbage having a high content of lycopene was used as the Kimchi cabbage, and two kinds of lactic acid bacteria derived from kimchi, the *Leuconostoc mesenteroides* CJLM119 and the *Lactobacillus plantarum* CJLP133 strains, which were isolated and identified in Example <1-1>, were added.

### <2-3> Preparation of concentrate of kimchi extract

The kimchi prepared in Examples <2-1> and <2-2> was freeze-dried and then ground into powders. To the powders, 20 times the weight of the powders of methanol was added, and then, extraction was performed with a stirrer for 12 hours. The extracted supernatant was filtered through a filter paper, and methanol was again added to the remaining precipitate to perform extraction for 12 hours. The extract was thermally concentrated at a temperature of about 40°C by a concentrator to prepare a concentrate, and the concentrate was stored at 4°C for use in further experiments.

### Example 3: Determination of growth inhibitory effect against Helicobacter pylori

### <3-1> Culture of Helicobacter pylori strain

*Helicobacter pylori* strain ATCC 43504 (American Type Culture Collection, a cagA+ and vacA s1-m1 type's strain) was cultured at 37°C in BBL Trypticase soy (TS) agar medium (TSAII; BD Biosciences, Franklin Lakes, NJ) with 5% sheep blood under microaerophilic condition (BD GasPaK EZ Gas Generating Systems, BD Biosciences) for 3 days. The *Helicobacter pylori* strain was harvested in TS medium, centrifuged at 3000 X g for 5 minutes, and resuspended in the medium at a final concentration of 10⁹ colony-forming units (CFUs)/mL. In all experiments, cultures grown for 72 hours in TS agar medium were used.

### <3-2> Determination of growth inhibitory activity against Helicobacter pylori

In order to determine the inhibitory effect of the kimchi of the present application against *Helicobacter pylori,* the medium plated with the *Helicobacter pylori* strain of Example <3-1> was treated and cultured with different concentrations of the concentrate of the kimchi extract prepared in Example <2-3>, and the size of the inhibition zone was measured.

As a result, it was found that the concentrate of the kimchi extract of the present application has an inhibitory effect against *Helicobacter pylori* bacteria at a concentration of 500 mg/ml or more (FIG. 1).

### Example 4: Determination of effects on Helicobacter pylori-associated disease in normal gastric cell line (RGM1; normal gastric mucosa cell)

### <4-1> Determination of inhibitory activity on angiogenetic factors

In order to determine the effect of the kimchi of the present application on vascular growth factors, normal gastric cell line RGM1 cells were treated with 50 MOI of *Helicobacter pylori* and then with the concentrate of the kimchi extract prepared in Example <2-3> at 5 mg/mL concentration for 6 hours. After treatment, media components were removed and cells were washed twice with PBS (Dulbecco) solution. Then, RiboEX (500 µl, GeneAll, Seoul, South Korea) was added to a plate incubated at 4°C for 10 minutes to perform RT-PCR. RiboEX was collected and placed into a 1.5 ml tube, and 100 µl of chloroform was added and mixed slowly. After 10 minutes of incubation on ice, samples were centrifuged at 10,000 g for 30 minutes. The supernatant was extracted and mixed with 200 µl of isopropanol and the mixture was incubated at 4°C for 1 hour. After centrifugation at 13,000 g for 30 minutes, the precipitate was washed with 70% (vol/vol) ethanol. Ethanol was completely evaporated, and then the pellet was dissolved in 100 µl of DEPC treated water (Invitrogen Life Technologies, Carlsbad, Calif.). Then PCR was performed to determine changes in related genes.

As a result, it was found that the expressions of VEGF and bFGF, which are indicators of angiogenesis factors increased by treatment with *Helicobacter pylori,* were reduced in cancer preventive kimchi treatment group (CPK) (FIG. 2).

### <4-2> Comparison of anti-inflammatory effect

In order to confirm the anti-inflammatory effect of the kimchi of the present application, RGM1 cells were treated with 50 MOI of *Helicobacter pylori* and then with the concentrate of the kimchi extract prepared in Example <2-3> at 5 mg/mL concentration. Then, changes in related genes were determined according to the RT-PCR method in Example <4-1>.

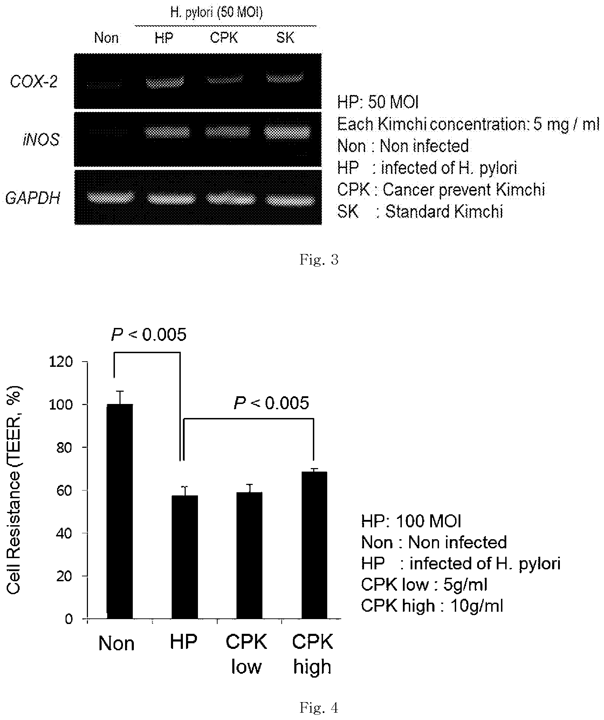

As a result, it was found that the expressions of COX-2 and iNOS, which are indicators of inflammation increased by treatment with *Helicobacter pylori,* were reduced in cancer preventive kimchi treatment group (CPK) (FIG. 3).

### <4-3> Comparison of cell junction effect

In order to determine the effect of the kimchi of the present application on indicators of the tight junctions of cells, RGM1 cells were treated with 100 MOI of *Helicobacter pylori* and then with the concentrate of the kimchi extract prepared in Example <2-3> at 5 g/mL and 10 g/mL concentrations for 6 hours. At this time, the upper part of the single cell layer, which became confluent due to the proliferation of cells, was washed three times with 0.5 ml of cold D-PBS(-) and filled with 0.5 ml of cold HBSS and transferred to a new well containing 1.5 ml of cold HBSS. After leaving the well as it is for 30 minutes, transepithelial electrical resistance (TERR%) between the upper and lower layers was measured.

As a result, it was found that in a *Helicobacter pylori* infection model, the tight junctions of the cells were restored when treating with the cancer preventive kimchi of the present application at a high concentration (FIG. 4).

### Example 5: Determination of effects on Helicobacter pylori-associated disease in gastric cancer cell line (MKN28; gastric cancer cell)

### <5-1> Determination of growth inhibitory effect on gastric cancer cell line

In order to determine the growth inhibitory effect of the kimchi of the present application on gastric cancer cell line, MKN28 cells were treated with the concentrates of the kimchi extracts prepared in Example <2-3> of the standard kimchi and the cancer preventive kimchi at concentrations of 10, 20, and 50 mg/ml for 6 hours, respectively. Then, the cell viability of each treatment group was compared.

As a result, it was found that the cancer preventive kimchi of the present application can more significantly inhibit the growth of the gastric cancer cell line in a concentration-dependent manner compared to the standard kimchi (FIG. 5).

### <5-2> Determination of apoptosis induction effect on gastric cancer cell line

In order to determine the apoptosis induction effect of the cancer preventive kimchi treatment in cancer cells having the characteristic of no apoptosis, each cell was treated with the concentrates of the kimchi extracts prepared by the method in Example <2-3> of the standard kimchi and the cancer preventive kimchi at an appropriate concentration for 48 hours, and then, was recovered with 1X PBS containing 1% FBS. AnnexinV & Dead Cell Reagent was dispensed at 1:1 to each cell recovered at a different concentration, and then, the cells were reacted for 20 minutes in a dark condition at room temperature, and the ratio of apoptosis-induced cells was measured and analyzed.

As a result, it was found that when treating MKN28 gastric cancer cell line with the cancer preventive kimchi of the present application, apoptosis was induced in 70% or more of the cells (FIG. 6).

### <5-3> Determination of apoptosis regulatory factors of gastric cancer cell line

In order to determine the effect of the kimchi of the present application on the apoptosis regulatory factors of gastric cancer cell line, MKN28 gastric cancer cell line was treated with the concentrate of the kimchi extract prepared in Example <2-3> at a concentration of 20 mg/ml, and then the medium components were removed, and the cells were washed twice with PBS (Dulbecco) solution. The cells were lysed with ice-cold cell lysis buffer (Cell Signaling Technology, Denver, MA) containing 1 mM PMSF (phenylmethylsulfonyl fluoride, Sigma Aldrich, St Louis, MO). After 20 minutes of the reaction, each sample was centrifuged at 10,000 x g for 10 minutes. Then, the supernatant was obtained. Proteins of the lysate were separated by sodium dodecyl sulfate-polyacrylamide gel electrophoresis (SDS-PAGE), transferred to a polyvinylidene fluoride (PVDF) membrane, reacted with the primary antibody, washed, and then reacted with the peroxidase-conjugated secondary antibody, washed again, and then changes in the expressions of apoptosis regulatory factors were determined by the ECL system (GE Healthcare, Buckinghamshire, UK).

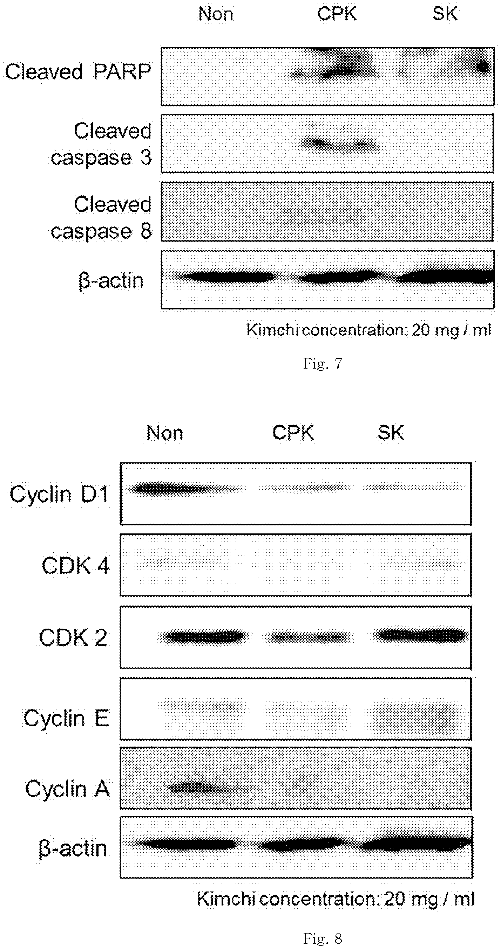

As a result, it was found that the expressions of cleaved caspase 3, cleaved caspase 8, and cleaved PARP, which are involved in the apoptosis, were remarkably increased in the cancer preventive kimchi of the present application-treatment group (CPK) as compared with those in the standard kimchi treatment group (SK) (FIG. 7).

### <5-4> Determination of cell cycle-related factors of gastric cancer cell line

In order to determine the effect of the kimchi of the present application on the cell cycle-related factors of gastric cancer cell line, MKN28 cancer cell line was treated with the concentrate of the kimchi extract prepared in Example <2-3> at a concentration of 20 mg/ml, and then the medium components were removed, and the cells were washed twice with PBS (Dulbecco) solution. The cells were lysed with ice-cold cell lysis buffer (Cell Signaling Technology, Denver, MA) containing 1 mM PMSF (Sigma Aldrich, St Louis, MO). After 20 minutes of the reaction, each sample was centrifuged at 10,000 x g for 10 minutes. Then, the supernatant was obtained. Proteins of the lysate were separated by SDS-PAGE, transferred to a PVDF membrane, reacted with the primary antibody, washed, and then reacted with the peroxidase-conjugated secondary antibody, washed again, and then changes in the expressions of cell cycle-related proteins were determined by the ECL system (GE Healthcare, Buckinghamshire, UK) .

As a result, the expressions of cyclin D1, CDK4, CDK2, cyclin E, and cyclin A, which are involved in cell cycle, were remarkably decreased in the cancer preventive kimchi of the present application-treatment group (CPK) as compared with those in the standard kimchi treatment group (SK). From this, it was found that the mechanism of apoptosis of the gastric cancer cell line is due to cell cycle regulation (FIG. 8) .

### Example 6: Determination of effect on Helicobacter pylori-associated disease in long-term model of animals infected with Helicobacter pylori

In order to determine the inhibitory effect of the kimchi of the present application on pathological parameters in a long-term model of animals infected with *Helicobacter pylori,* five-week-old female C57BL/6 mice (Charles River Japan) were fed sterilized commercial pellet diets (Biogenomics, South Korea) and sterile water ad libitum, and housed in a conditioned room at a temperature of 24°C. After 1 week of adaptation, 20 mg/kg pantoprazole was injected three times to facilitate *Helicobacter pylori* colonization through lowered gastric acidity. And then each animal was intragastrically inoculated with a suspension of *Helicobacter pylori* (containing 10⁹ CFUs/mL) with an equal volume (0.3 mL) of clean TS broth using gastric intubation needles. Control group was given injections of clean TS broth, and all experimental groups were given injections of *Helicobacter pylori* total four times within a week. The mice were fed a special pellet diet based on AIN76 containing 7.5% NaCl to cause gastritis for 4 weeks.

Then, *Helicobacter pylori* positive mice were randomly divided into four groups (n = 10). Pellet diet AIN76 containing 7.5% NaCl were administrated for 24 weeks to promote *Helicobacter pylori-induced* carcinogenic process in all infected animals. Control group and experimental groups are shown in FIG. 9, respectively. Group 1: Control group, Group 2: *Helicobacter* pylori-infected group, Group 3: *Helicobacter* pylori-infected and cancer preventive kimchi low concentration diet group, Group 4: *Helicobacter pylori-*infected and cancer preventive kimchi high concentration diet group, Group 5: *Helicobacter* pylori-infected and standard kimchi diet group were experimented, respectively.

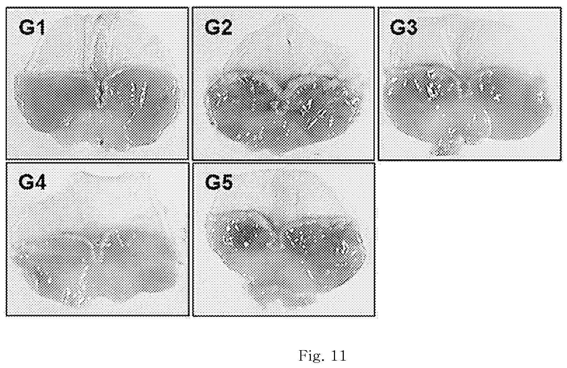

As results, the gross lesion index was considerably increased in the control group G2, whereas significantly decreased in the cancer preventive kimchi-treated groups G3 and G4 (p<0.01). In addition, a considerable size of gastric cancer was formed in the control group G2, whereas the formation of gastric cancer was decreased in the cancer preventive kimchi-treated groups G3 and G4 (FIGS. 10 and 11).

Although exemplary embodiments of the present application have been described above, the scope of the present application is not limited to the specific embodiments as described above, a person skilled in the art can change the present application within the scope described in the claims of the present application.

## Claims

1. Kimchi prepared by salting and seasoning vegetables, and then fermenting the same with lactic acid bacteria, wherein the lactic acid bacteria comprises *Leuconostoc mesenteroides* CJLM119 (Accession number: KCTC 11403BP) and *Lactobacillus plantarum* CJLP133 (Accession number: KCTC 13043BP).

2. The kimchi of claim 1, wherein the vegetables are selected from the group consisting of Kimchi cabbages, white radishes, spring onions, mustard leaves, cucumbers, and combinations thereof.

3. The kimchi of claim 2, wherein the vegetables are Kimchi cabbages.

4. The kimchi of claim 3, wherein the Kimchi cabbages are those with a high lycopene content.

5. The kimchi of claim 4, wherein the lycopene is contained in an amount of 0.05 to 5 mg per 100 g of the Kimchi cabbages.

6. The kimchi of claim 1, wherein the *Leuconostoc mesenteroides* CJLM119 is contained in 0.01 to 5 wt% based on the total weight of the kimchi and the *Lactobacillus plantarum* CJLP133 is contained in 0.01 to 5 wt% based on the total weight of the kimchi.

7. A composition for ameliorating a *Helicobacter* pylori-associated disease, the composition comprising the kimchi of any of claims 1 to 6 or an extract thereof.

8. The composition of claim 7, wherein the kimchi extract is an extract obtained by extracting kimchi with water, an organic solvent or a mixed solvent thereof.

9. The composition of claim 8, wherein the organic solvent is selected from the group consisting of alcohols, hexane, ethers, glycerol, propylene glycol, butylene glycol, ethyl acetate, methyl acetate, dichloromethane, chloroform, benzene and mixed solvents thereof.

10. The composition of claim 8, wherein the mixed solvent is selected from the group consisting of water, a C₁-C₄ alcohol, and a mixed solvent of the C₁-C₄ alcohol and water.

11. The composition of claim 10, wherein the mixed solvent is an aqueous methanol solution or an aqueous ethanol solution.

12. The composition of claim 7, wherein the kimchi extract is selected from the group consisting of an extract, a concentrate of the extract, a dried product of the concentrate, and powders.
